# EUROPEAN PATENT SPECIFICATION

(11) **EP 3 697 461 B1**
(45) Date of publication and mention of the grant of the patent: **24.12.2025**
(21) Application number: 18795739.4
(22) Date of filing: 17.10.2018
(51) Int. Cl.: A61L 27/38, A61L 27/54, A61L 27/56

(54) **POROUS MATERIAL**
PORÖSES MATERIAL
MATÉRIAU POREUX

(30) Priority: 18.10.2017 GB 201717112
(43) Date of publication of application: 26.08.2020
(73) Proprietor: The University Of Nottingham, Nottingham, Nottinghamshire NG7 2RD (GB)
(72) Inventor: ROBERTS, George, Nottingham Nottinghamshire NG7 2RD (GB); GRANT, David, Nottingham Nottinghamshire NG7 2RD (GB); FELFEL, Reda, Nottingham Nottinghamshire NG7 2RD (GB); SCOTCHFORD, Colin, Nottingham Nottinghamshire NG7 2RD (GB); SOTTILE, Virginie, Nottingham Nottinghamshire NG7 2RD (GB)
(74) Representative: Barker Brettell LLP
(86) International application number: PCT/GB2018/052992
(87) International publication number: WO 2019/077348

(56) References cited:
- EP-A1- 1 273 615
- WO-A1-03/077964
- WO-A1-97/24090
- US-A1- 2005 137 272

## Description

The present invention relates to a porous material and articles or products comprising the porous material. The invention also relates to articles or products comprising the porous material.

Porous materials are useful in a wide range of applications, including medical and non-medical applications.

An example of a non-medical application is use of porous materials in filtration and/or purification of fluids, e.g. water.

Biocompatible porous materials can be used in biomedical implants, e.g. for controlled release of a drug or other therapeutic agent, or as a scaffold to encourage cell and tissue, e.g. bone, ingrowth, regrowth and/or regeneration.

Orthopaedic pathologies such as osteoarthritis have a large incidence, a high socio-economic cost and a major impact on quality of life for sufferers.

The traumatic injury to, and osteoarthritic degeneration of, the articular cartilage surface and bone (e.g. in the knee or other joints) ultimately results in catastrophic destruction of the joint surface and chronic pain, eventually leading to Total Joint Replacement - an invasive, debilitating and costly procedure.

Early interventions to treat moderate/painful osteoarthritis include Minimally Invasive Surgery, microfracture and orthobiological scaffold-based implants.

Implants could offer better long-term outcomes than alternatives. However, current products face delamination, cost-effectiveness issues, and/or collagen supply limitations.

Typical existing commercial orthobiologic implants (e.g. Biomatrix, Chondro-gide, and Maioregen) are collagen-based, have high unit prices and limited clinical efficacy. US 2005/137272 discloses a dried gelled foam comprising a gelling agent.

Examples of known polymeric scaffolds for use in treating bone and/or cartilage degeneration are disclosed in US20140012393A1, US20140219962A1, KR20160001071A and KR101333381B1.

Although joint repair remains problematic because of its complex structure, innovative strategies for articular repair have been proposed. However, many subsequently fail as secure construct fixation to the osteochondral bone often cannot be achieved or maintained under load-bearing conditions.

The present invention has been conceived with the foregoing problems in mind.

The present invention relates to a porous material comprising a substantially continuous matrix comprising an at least partially cross-linked water-soluble polymer with pores therein and a plurality of regions of differing pore sizes, average pore sizes, pore size distributions and/or porosities, wherein each region has a desired porosity including a desired pore density and/or pore size distribution..

The porous material may be a biomaterial. A generally accepted definition of a biomaterial is a non-viable substance that has been engineered to take a form which, alone or as part of a complex system, is used to direct, by control of interactions with components of living systems, the course of any therapeutic or diagnostic procedure (see, for instance, https://www.journals.elsevier.com/biomaterials).

The porous material may be biocompatible. The term biocompatible refers to the capability of a material to coexist with living tissues or organisms without causing harm, e.g. by not being toxic, injurious, or physiologically reactive and not causing immunological rejection. Biocompatibilty is an important attribute for biomaterials, e.g. when used in surgical implants.

The porous material may be bioresorbable. A bioresorbable material is one that can be broken down and absorbed in the body.

The water-soluble polymer may comprise a biopolymer. Biopolymers are polymers produced by living organisms. Typically, the biopolymer may include a protein or a polysaccharide.

The water-soluble polymer may comprise chitosan, collagen or hyaluronic acid.

Other examples of suitable water-soluble polymers may include: gelatine, agarose, alginate, polyvinyl alcohol, dextran, polyacrylic acid, polyethylene glycol, polyvinyl pyrrolidone, polyacrylamides, cellulose ethers, starch or starch-based derivatives.

The non-water-soluble porogen may comprise substantially spherical bodies, e.g. microspheres. The non-water-soluble porogen may comprise non-spherical bodies and/or other easy-to-produce geometries such as irregular ground particles, cylinders, and fibres or bodies made up of regular or irregular agglomerations of smaller particles.

The non-water-soluble porogen may comprise a polymer such as polycaprolactone and/or polylactic acid.

The bodies of non-water soluble porogen, e.g. substantially spherical bodies such as microspheres, may have a specific size distribution selected based on the desired pore size for the porous material being produced.

For instance, the bodies, e.g. substantially spherical bodies such as microspheres, may have a longest dimension, e.g. diameter, of up to or at least 100 µm, up to or at least 150 µm, up to or at least 180 µm, up to or at least 200 µm , up to or at least 250 µm, up to or at least 300 µm, up to or at least 350 µm, up to or at least 400 µm, up to or at least 425 µm, up to or at least 450 µm and/or up to or at least 500 µm.

Through appropriate selection of the porogen, good control of the final porosity, pore topography, average pore size and/or pore size distribution of the material may be achieved.

In embodiments, the pores may be generally spherical in form.

The pores, may have a longest dimension, e.g. diameter, of up to or at least 100 µm, up to or at least 150 µm, up to or at least 180 µm, up to or at least 200 µm , up to or at least 250 µm, up to or at least 300 µm, up to or at least 350 µm, up to or at least 400 µm, up to or at least 425 µm, up to or at least 450 µm and/or up to or at least 500 µm.

The cross-linking agent may be water-soluble. For instance, the cross-linking agent may comprise glutaraldehyde, formaldehyde or genipin.

In some embodiments, use of a natural cross-linking agent such as genipin may be preferred, due to its low toxicity, good biocompatibility and/or the good stability of the crosslinks.

Genipin may be a particularly suitable cross-linking agent in embodiments in which the water-soluble polymer comprises chitosan.

In embodiments, in which genipin is used as the cross-linking agent, e.g. with chitosan as the water-soluble polymer, the method may comprise a step of washing the material in a solution comprising a reducing agent. The solution may be an aqueous solution. An example of a suitable reducing agent may be sodium borohydride (NaBH₄).

Advantageously, washing the material in a solution comprising a reducing agent such as NaBH₄ has been found to be effective in removing the blue/purple colour introduced to the material by the genipin crosslinking with little or no effect on the mechanical and/or biological properties of the porous material. The reducing agent may comprise a natural reducing agent.

Two or more of the water-soluble polymer, the non-water-soluble porogen and the cross-linking agent may be mixed together prior to being placed in the mould.

Performing the cross-linking reaction may comprise initiating the cross-linking reaction. The cross-linking reaction typically will then propagate and terminate.

The cross-linking reaction may be performed substantially to, or at least partially towards, completion, i.e. when there is essentially no more free cross-linking agent available and/or when there is essentially no more free non-cross-linked water-soluble polymer available.

The way in which the cross-linking reaction is initiated will depend on the given combination of water-soluble polymer and cross-linking agent. For instance, in embodiments where the water-soluble polymer comprises chitosan and the cross-linking agent comprises genipin, the cross-linking reaction may be initiated by adjusting the pH. A buffer solution may be used to adjust the pH. A suitable pH range for initiating the cross-linking reaction between chitosan and genipin may be at least 1 and/or up to 8. The pH may be up to or at least 2, up to or at least 2.5, up to or at least 3, up to or at least 4, up to or at least 5, up to or at least 6 and/or up to or at least 7. The cross-linking reaction between chitosan and genipin may proceed well at a pH of approximately 7.4.

Initiating the cross-linking reaction may comprise imparting energy, e.g. in the form of heat and/or electromagnetic radiation such as ultraviolet light.

The cross-linking agent may comprise a chemical cross-linking agent or a physical cross-linking agent.

Chemical cross-linking agents may include glutaraldehyde, genipin, glyoxal, dextran sulfate, 1,1,3,3-tetramethoxypropane, oxidized cylclodextrins, ethylene glycoldiglyceryl ether, ethylene glycol diglycidyl ether (EGDE), and diisocyanate.

Physical cross-linking agents, e.g. of chitosan, may include pentasodium tripolyphosphate (TPP), β-glycerol phosphate, sodium polyphosphate and calcium chloride.

Radiation- and photo-cross-linking may be used.

Leaching out the porogen may comprise use of an alkaline alcohol solution at an elevated temperature. For instance, the alkaline may comprise sodium hydroxide or potassium hydroxide. The alcohol may comprise ethanol or methanol. The elevated temperature may be approximately 50°C. The material may be exposed to, e.g. immersed in, the alkaline alcohol solution at an elevated temperature for a predetermined period of time. The predetermined period of time may be up to or at least 5 minutes, up to or at least 15 minutes, up to or at least 30 minutes, up to or least 1 hour, up to or at least 2 hours, up to or at least 3 hours, up to or at least 4 hours, up to or at least 5 hours, and/or up to or at least 6 hours. The process of leaching out the porogen may be performed at room temperature. The process of leaching out the porogen may be performed at lower or higher temperature, e.g. lower or higher than room temperature, but it will require shorter or longer times dependent on the temperature calculable by an Arrhenius relationship.

In embodiments, steps (ii) to (v) of the method may be repeated on one or more occasions to form one or more further layers. Any given further layer, may overlap at least partially one or more of the previous layers. Each further layer may be formed on top of previous layer(s). Accordingly, the material may be built up in a layer by layer fashion.

Any given further layer may be integral at least partially to one or more of the previous layers. A continuous non-delaminating interface may be formed between the layers.

In each layer, a desired porosity can be obtained by selecting a desired relative amount of porogen and/or a porogen having a desired size distribution.

Spatial variations in porosity, pore size distribution and/or average pore size may be achieved by changing the relative amount of the porogen, the size distribution of the porogen and/or the average particle size of the porogen each time that steps (ii) to (v) are repeated. The spatial variation(s) may be predetermined.

Hence, for example, the porous material may have graded porosity.

In embodiments where the porous material is a biomaterial, the predetermined, e.g. graded, porosity may be selected to mimic the structure and organisation of native tissue. Hence, for instance, mechanical shielding effects may be minimised and/or tissue ingrowth may be promoted. Selection of specific pore sizes may promote desired phenotypic expression leading to specific tissue formation.

The porous material, or one or more regions, e.g. layers, within the porous material, may have a porosity of at least 10%, at least 20% and/or up to 70%, up to 80% or up to 90%.

In an embodiment, the intermediate material may have the form of a gel.

In an embodiment, at least a portion of the material may comprise one or more functional additives. For instance, the material may comprise an additive for promoting osteoconductivity such as a compound containing calcium phosphate, e.g. tricalcium phosphate, hydroxyapatite or chondroitin sulphate.

In the portion(s) of the material comprising the one or more functional additives, e,g. tricalcium phosphate or hydroxyapatite, the one or more functional additives may be present in an amount of up to or at least 10 wt%, up to or at least 20 wt%, up to or at least 30 wt%, up to or at least 40 wt%, up to or at least 50 wt%, up to or at least 60 wt%, up to or at least 70 wt%, up to or at least 80 wt% and/or up to or at least 90 wt%.

The one or more functional additives may comprise microparticles or nanoparticles.

In an embodiment, the method may comprise a preliminary step of designing the material.

When dry, the material may have a compressive strength of up to or at least 0.05 MPa, up to or at least 0.1 MPa or up to or at least 0.2 MPa. When dry, the material may have a compressive modulus of up to or at least 0.5 MPa, up to or at least 1 MPa up to or at least 2 MPa or up to or at least 5 MPa.

When wet, the material may have a compressive strength of up to or at least 2 kPa, up to or at least 5 kPa or up to or at least 10 kPa. When wet, the material may have a compressive modulus of up to or at least 12 kPa, up to or at least 25 kPa, up to or at least 50 kPa or up to or at least 500 kPa.

The material may be manufactured to any shape by choosing a suitable mould. The shape of the mould may correspond with the shape of an article comprising the porous material.

In an embodiment, the method may comprise a further step of machining, e.g. cutting, a piece of the porous material to form the article.

In an embodiment, the article may be an article for use in a biomedical application. The article may be a medical device or a portion thereof. For instance, the article may be a scaffold for osteochondral repair, a scaffold for bone grafting, a scaffold for cartilage repair/reconstruction, an implant, e.g. for drug delivery, or a vascular stent or tube.

At least some of the pores may be loaded with a drug or other therapeutic agent. In an embodiment, at least some of the pores may contain cells, e.g. stem cells.

At least a portion of the article may be coated and/or impregnated with one or more antimicrobial agents.

At least a portion of the article may be coated and/or impregnated with one or more biological agents, which may include for example collagen, glycans, fibronectin, platelet lysate. The biological agent(s) may promote cellular response to the article.

An aspect provides a porous material comprising a substantially continuous matrix comprising an at-least-partially-cross-linked water-soluble polymer with pores therein and a plurality of regions, e.g. layers, of differing pore size, average pore sizes, pore size distributions and/or porosities, wherein each region has a desired porosity, including a desired pore density and/or pore size distribution.

The porous material may be a biomaterial. The porous material may be biocompatible. The porous material may be bioresorbable.

The water-soluble polymer may comprise a biopolymer. Typically, the biopolymer may include a protein or a polysaccharide.

The water-soluble polymer may comprise chitosan, collagen or hyaluronic acid.

The term "chitosan" as used in this disclosure may not refer to a precise chemical structure but to a continuum of copolymers of N-acetyl-D-glucosamine and D-glucosamine residues, having 0-75% of the residues present as N-acetyl-D-glucosamine, and being soluble in dilute aqueous acid. Such chitosan may be obtained by deacetylation of chitin, by re-N-acetylation of a more highly deacetylated chitosan, or directly from some fungi.

Other examples of suitable water-soluble polymers may include: collagen, gelatine, agarose, alginate, polyvinyl alcohol, dextran, polyacrylic acid, polyethylene glycol, polyvinyl pyrrolidone, polyacrylamides, cellulose ethers, starch or starch-based derivatives.

In embodiments, the pores may be generally spherical in form.

The pores, may have a longest dimension, e.g. diameter, of up to or at least 100 µm, up to or at least 150 µm, up to or at least 180 µm, up to or at least 200 µm , up to or at least 250 µm, up to or at least 300 µm, up to or at least 350 µm, up to or at least 400 µm, up to or at least 425 µm, up to or at least 450 µm and/or up to or at least 500 µm.

The cross-linking of the water-soluble polymer may be provided by a cross-linking agent. The cross-linking agent may comprise glutaraldehyde, formaldehyde or genipin.

The cross-linking agent may comprise a chemical cross-linking agent or a physical cross-linking agent.

Chemical cross-linking agents may include glutaraldehyde, genipin, glyoxal, dextran sulfate, 1,1,3,3-tetramethoxypropane, oxidized cylclodextrins, ethylene glycoldiglyceryl ether, ethylene glycol diglycidyl ether (EGDE), and diisocyanate.

Physical cross-linking agents, e.g. of chitosan, may include pentasodium tripolyphosphate (TPP), β-glycerol phosphate, sodium polyphosphate and calcium chloride.

Radiation- and photo-cross-linking may be used.

In some embodiments, use of a natural cross-linking agent such as genipin may be preferred, due to its low toxicity, good biocompatibility and/or the good stability of the crosslinks.

Genipin may be a particularly suitable cross-linking agent in embodiments in which the water-soluble polymer comprises chitosan.

In embodiments, the material may be substantially colourless. The material may be white or off-white in colour. Alternatively, the material may be coloured, e.g. blue or purple, at least in part.

The plurality of regions, e.g. layers, may have no mechanical weakness between them.

Any given region, e.g. layer, may overlap at least partially one or more other regions.

The material may comprise spatial variations in porosity. The spatial variations in porosity may be predetermined.

The spatial variations in porosity may arise from variations in pore density and/or pore size distribution within the material. For instance, the or each region or layer may have a different pore density and/or pore size distribution.

In embodiments, the porous material may have graded porosity.

In embodiments where the porous material is a biomaterial, the spatial variation in porosity, e.g. graded porosity, may be selected to mimic the structure and organisation of native tissue. The spatial variation in porosity may be predetermined. Hence, for instance, in use, mechanical shielding effects may be minimised and/or tissue ingrowth may be promoted.

The porous material, or one or more regions, e.g. layers, within the porous material, may have a porosity of at least 10%, at least 20% and/or up to 70%, up to 80% or up to 90%.

In an embodiment, at least a portion of the material may comprise one or more functional additives. For instance, the material may comprise an additive for promoting osteoconductivity such as a compound containing calcium phosphate, e.g. tricalcium phosphate, hydroxyapatite or chondroitin sulphate.

In an embodiment, the material may be resilient. The material may be capable of returning to its original shape after being subjected to up to 40%, up to 50%, up to 70% or up to 80% or more compressive strain.

When dry, the material may have a compressive strength of up to or at least 0.05 MPa, up to or at least 0.1 MPa or up to or at least 0.2 MPa. When dry, the material may have a compressive modulus of up to or at least 0.5 MPa, up to or at least 1 MPa, up to or at least 2 MPa or up to or at least 5 MPa.

When wet, the material may have a compressive strength of up to or at least 2 kPa, up to or at least 5 kPa or up to or at least 10 kPa. When wet, the material may have a compressive modulus of up to or at least 12 kPa, up to or at least 25 kPa, up to or at least 50 kPa or up to or at least 500 kPa.

Another aspect provides an article comprising the porous material of the preceding aspect of the invention.

In an embodiment, the article may be an article for use in a biomedical application. The article may be a medical device or a portion thereof. For instance, the article may be a scaffold for osteochondral repair, a scaffold for bone grafting, an implant, e.g. for drug delivery, or a vascular stent or tube.

At least some of the pores may be loaded with a drug or other therapeutic agent. In an embodiment, at least some of the pores may contain stem cells.

At least a portion of the article may be coated and/or impregnated with one or more antimicrobial agents.

At least a portion of the article may be coated and/or impregnated with one or more biological agents, which may include for example collagen, glycans, fibronectin, platelet lysate. The biological agent(s) may promote cellular response to the article.

At least a portion of the article may be coated and/or impregnated with one or more metals or metal ions, e.g. Mg²⁺. Metal ions such as Mg²⁺ may promote cell function.

In embodiments, the article may be adapted for a non-biomedical use. For instance, the article may be adapted for use in filtration and/or purification of a fluid, e.g. water.

In order that the invention can be well understood, it will now be described, by way of example only, with reference to the accompanying drawings, in which:
Figure 1 is a cross-section through a portion of native tissue;
Figure 2 shows a scaffold for osteochondral repair according to an embodiment;
Figure 3 is a scanning electron microscope (SEM) image of a cross section of a scaffold for osteochondral repair;
Figure 4 is an SEM image of a bottom portion of the scaffold shown in Figure 2;
Figure 5 shows a scaffold for osteochondral repair according to an embodiment of the invention after *in vitro* mechanical testing;
Figure 6 is a graph showing results from compression testing of a scaffold for osteochondral repair according to an embodiment of the invention;
Figure 7 is a graph showing degradation behaviour of a scaffold for osteochondral repair according to an embodiment of the invention;
Figures 8 to 12 are fluorescent microscope images showing the progress of an *in vitro* cytocompatibility test carried out on a scaffold for osteochondral repair according to an embodiment of the invention;
Figure 13 is a scanning electron microscope (SEM) image of a scaffold for osteochondral repair according to an embodiment of the invention after a cytocompatibility test; and
Figure 14 is a graph showing the results of cell metabolic activity analysis carried out during a cytocompatibility test of a scaffold for osteochondral repair according to an embodiment of the invention.

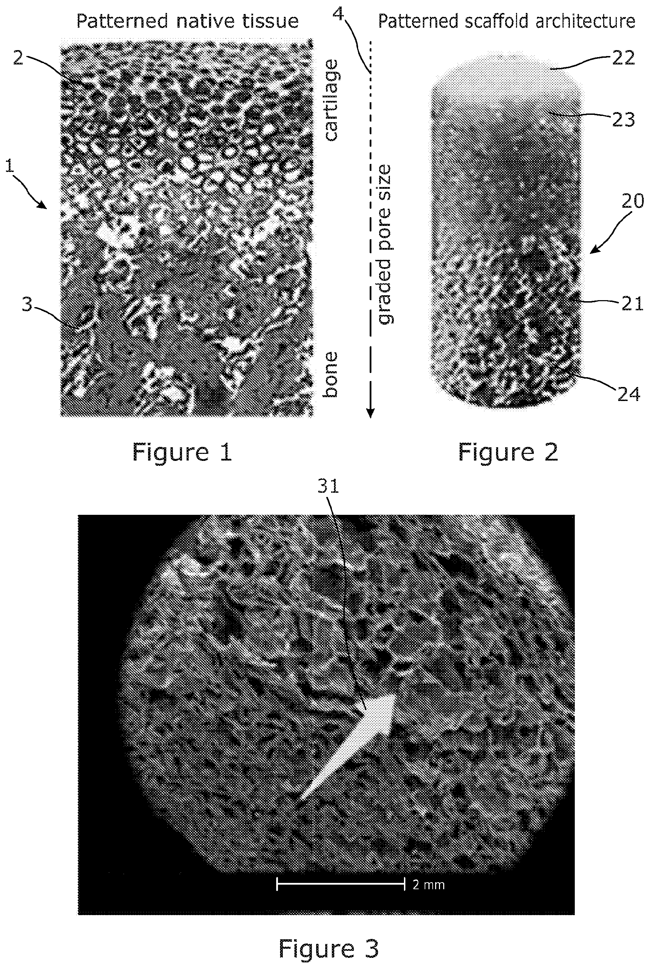

Figure 1 shows a cross-section through a portion of patterned native tissue 1 from an end portion of a bone at a joint such as a knee. The end portion of the bone comprises a region of cartilage 2 and a region of bone tissue 3. The patterned native tissue 1 has a graded porous structure, in which average pore size increases in the direction indicated by dashed arrow 4. The pores in the region of cartilage 2 are smaller than the pores in the region of bone tissue 3.

Porous scaffolds or constructs, intended for use in osteochondral repair, according to the invention were manufactured generally as follows.

Chitosan was mixed with genipin, polycaprolactone microspheres and hydroxyapatite nanoparticles to form a first mixture. The polycaprolactone microspheres had diameters in the range of from 180 to 425 µm. The first mixture was placed into a mould thereby providing a first layer of material.

Chitosan was mixed with genipin and polycaprolactone microspheres to form a second mixture. The polycaprolactone microspheres in the second mixture had a smaller average diameter than the polycaprolactone microspheres in the first mixture. The second mixture was placed into the mould on top of the first layer of material.

The pH was then adjusted through use of a buffer solution, in order to initiate a cross-linking reaction between the chitosan and the genipin.

Hence, an intermediate material having the form of a gel was formed, in which the cross-linked chitosan formed a substantially continuous matrix. The polycaprolactone microspheres were distributed within the matrix in two layers (corresponding to the first and second layers of material in the mould). In embodiments, there may be some intermingling of the two layers of microspheres at the interface between the first and second layers of material. Such intermingling may be unavoidable and/or may be desirable.

Water was removed from the intermediate material in the frozen state using freeze drying. Alternatively, the water could be removed from the intermediate material in the frozen state using a solvent phase separation technique.

After removal of the water, the polycaprolactone microspheres were removed by leaching out. The leaching out was effected by treating the intermediate material with an alkaline alcohol solution (e.g. potassium hydroxide and ethanol or potassium hydroxide and methanol) at 50°C.

The blue/purple colour caused by the genipin cross-linking was removed by washing the material in an aqueous solution comprising NaBH₄.

The porous material was then cut into a plurality of scaffolds (bilayer scaffolds), each scaffold containing the two layers with differing average pore sizes. The layer with the smaller pores (the second layer) was designed to mimic the structure of articular cartilage tissue. The layer with the larger pores (the first layer) was designed to mimic the structure of bone tissue. The second layer contained 70 wt% of hydroxyapatite.

An example of a methodology for making bilayer scaffolds will now be described.

The bilayer scaffolds were made by a combination of freeze gelation and particle leaching. Two different cross-linkers were used, glutaraldehyde and genipin to make two different scaffolds. A 1% solution of genipin was made by dissolving 0.5 g genipin in 50 ml ethanol and a 0.5 % solution of glutaraldehyde was made by diluting a 25% stock solution in distilled water. The bilayer structure was built up by making one layer first and then just before that set adding the second layer. Each layer of the scaffold was made by adding 4 ml of the cross-linker to 20 g gelled chitosan solution and 10 g porogen (polycaprolactone (PCL) microspheres) at a specific particle size range. The first layer was made using PCL microspheres at a particle size range of from 300 µm to 425 µm, placed into a mould and allowed to pre-crosslink at room temperature for approximately 2 hours. Then a second layer was made using a smaller PCL particle size range of from 180 µm to 300 µm microspheres and carefully poured on top. The structure was allowed to completely crosslink overnight in a sealed container to avoid dehydration, before it was covered and stored at -20 °C overnight. Two methods were conducted for water removal from the construct; freeze drying at - 100 °C or solvent phase separation in a pre-cooled (-20 °C) 1:1 solution of ethanol and aqueous ammonia. The composite structure was inserted into ammonia/ethanol mixture and stored at -20 °C for 48 hours in an airtight container. It was then washed copiously with ethanol and allowed to dehydrate slowly in ethanol for 48 hours at room temperature. Individual scaffolds produced either by freeze drying or solvent phase separation method were cut from the mould using a cork-borer and immersed in 2.5% potassium hydroxide solution for 4 hours at 50 °C to remove any porogen microspheres. The final processing stage involved immersion in 1 % sodium borohydride solution for 24 hours to remove any cytotoxic free amine groups and also the dark blue/purple colour resulting from the genipin cross-linking agent. Finally, the scaffolds were left to dry in a fume hood overnight.

Figure 2 shows a chitosan-based scaffold 20 for osteochondral repair according to an embodiment of the invention. The scaffold 20 was manufactured as described above. The scaffold 20 comprises a porous material according to the invention. The scaffold 20 comprises a generally cylindrical body 21 with a first cylinder end 22. Each cylinder end may be substantially flat or uneven or may be at least partially curved, e.g. dome-shaped, depending for example on the shape of the mould used in the manufacture of the scaffold. The porosity within the scaffold 20 is graded from a smaller average pore size to a larger average pore size in the direction indicated by dashed arrow 4. Accordingly, a first portion of the porous material 23 proximal to the first cylinder end 22 has relatively small pores, while a second portion of the porous material 24 distal from the first cylinder end 22 has relatively large pores. Between the first portion of the porous material 23 and the second portion of the porous material 24, the scaffold 20 is configured such that average pore size increases with distance from the first cylinder end 22. Accordingly, the scaffold 20 has a graded porous structure designed to mimic the patterned organisation of the native tissue (e.g. as illustrated in Figure 1).

Typically, a material for promoting bone ingrowth or osteoconductivity may be incorporated into the scaffold, particularly into the portion(s) of the scaffold that are designed to mimic bone tissue (e.g. the second portion of porous material 24 in the scaffold 20 shown in Figure 2). The material for promoting bone ingrowth may comprise calcium phosphate. The material for promoting bone ingrowth may comprise hydroxyapatite or tricalcium phosphate or other ceramic or glass phases with osteoconductivity or osteinductivity. The material for promoting bone ingrowth may be incorporated into the scaffold in the form of nanoparticles or microparticles.

Figure 3 is an SEM image of a cross section of a scaffold for osteochondral repair. A graded pore structure can be seen, with an arrow 31 indicating the transition from smaller pores to larger pores.

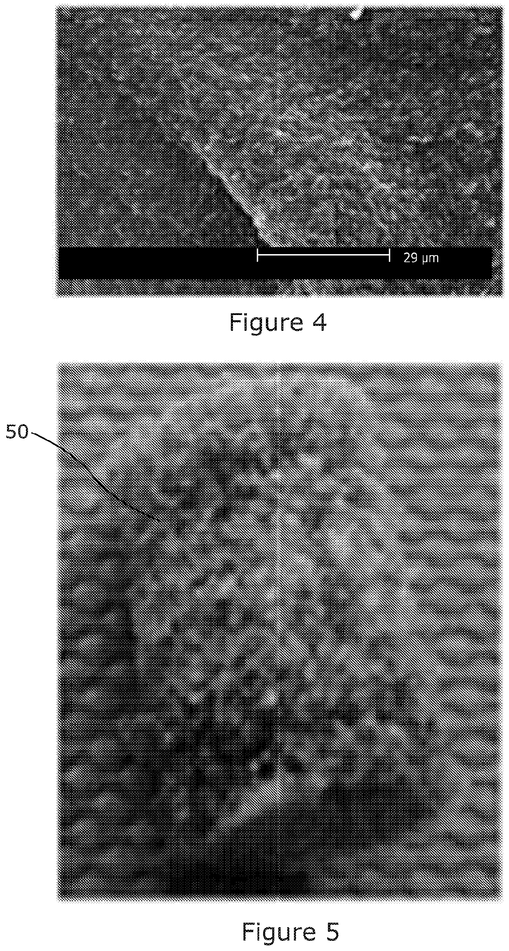

Figure 4 is an SEM image of a portion of the scaffold 20 distal from the first cylinder end 22. Hydroxyapatite nanoparticles have been incorporated into this portion of the scaffold 20 to promote osteoconductivity.

Figure 5 shows a chitosan-based scaffold 50 for osteochondral repair according to an embodiment of the invention. The scaffold 50 was manufactured as described above. The scaffold 50 has undergone *in vitro* tensile testing. As is immediately apparent from Figure 5, the scaffold 50 has retained its structural integrity.

By way of comparison, a commercially available multilayer collagen-based scaffold was considered. The scaffold failed by delamination at the interface between a first layer and a second layer of the scaffold. Failure occurred during cutting before any mechanical testing could be carried out. The delamination of the scaffold during cutting before any mechanical testing could be carried out is in stark contrast to the retained integrity of the scaffold 50 after mechanical testing.

Mechanical resilience of scaffolds according to embodiments of the invention has been tested extensively.

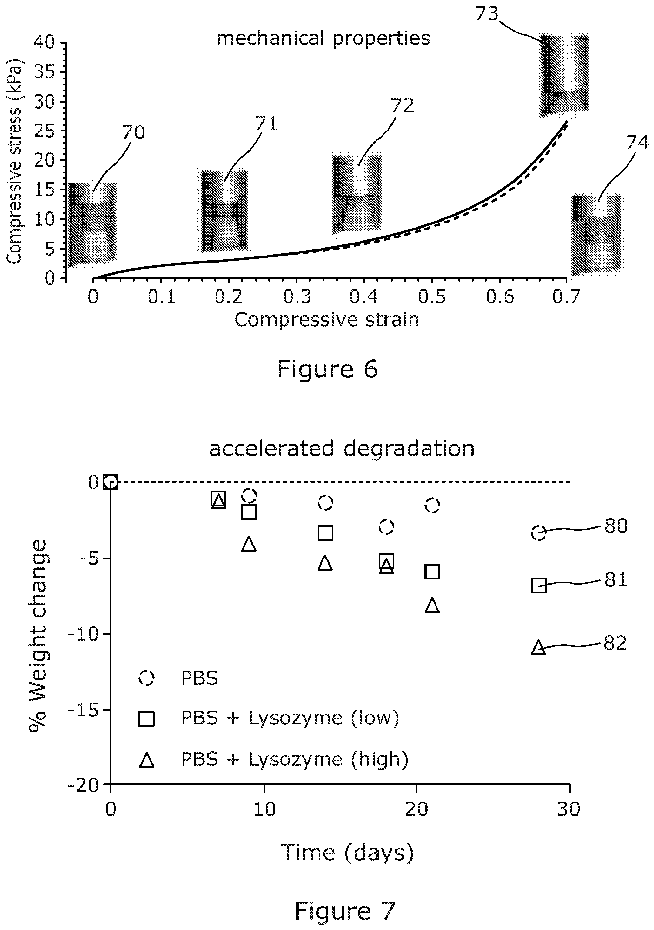

Figure 6 is a graph showing the results of a compression test carried out on a chitosan-based scaffold according to an embodiment of the invention. The y-axis is compressive stress (kPa) and the x-axis is compressive strain. The test was carried out up to 70% stain (i.e. 0.7 on the x-axis).

Figure 6 includes a series of photographs of the scaffold as the compression test progressed. A first photograph 70 shows the scaffold in its uncompressed state prior to commencement of the compression test. A second photograph 71 shows the scaffold deformed at around 20% compressive strain. A third photograph 72 shows the scaffold deformed at around 40% compressive strain. A fourth photograph 73 shows the scaffold deformed at around 70% compressive strain. A fifth photograph 74 shows the scaffold after the compression test had been completed. The scaffold returned to substantially its original size and shape, as illustrated by a comparison between the fifth photograph 74 and the first photograph 70 in Figure 6.

Furthermore, the tests showed no significant difference between successive compression cycles up to 70% strain. No permanent damage to the scaffold occurred during the compression test, and the scaffold fully recovered.

Mechanical testing using compression, tension, and recovery parameters has confirmed that the compressive strength and modulus for the bilayer scaffolds are approximately 0.1 and 1 MPa (for dry scaffolds) and 5 and 25 kPa (for wet scaffolds). The dry scaffold demonstrated similar elastic modulus to that of the native cartilage tissue (0.45-0.9 MPa). Tensile tests were conducted on the bilayer scaffolds to examine their delamination resistance at the interface between the layers. Failure tensile strength of the scaffolds was approximately 0.4 MPa and no delamination mode was seen, as failure occurred within the layers. The scaffold showed instant recovery after compressing up to approximately 80% strain, which advantageously may allow the arthroscopic implantation of the scaffold. Under wet conditions, the scaffold has sufficient mechanical strength to withstand handling and implantation stresses while maintaining its integrity. Unlike most multiphasic products on the market, the new scaffold material can easily be cut to size by the surgeon, which is an important asset for clinical use.

The degradation under *in vitro* conditions of scaffolds according to embodiments of the invention has also been tested extensively. The results of these tests have confirmed that the scaffolds degrade progressively over time.

Figure 7 is a graph showing the results of an *in vitro* degradation test. The y-axis is percentage weight change. The x-axis is time, measured in days. The duration of the test was 28 days.

The test was carried out on a chitosan-based scaffold according to an embodiment of the invention under three different conditions:
(i) in phosphate buffer solution (PBS) only, with the results being shown by a series of circular data points 80;
(ii) in PBS plus a low concentration of lysozyme, with the results being shown by a series of square data points 81; and
(iii) in PBS plus a high concentration of lysozyme, with the results being shown by a series of triangular data points 82.

Extensive cytocompatibility testing has also been carried out using *in vitro* cultures. The cytocompatibility testing demonstrated that the scaffold could support the attachment, growth and colonisation of human mesenchymal stem cells. Using specific medium conditions (France et al. 2014), successful culture and differentiation using a perfusion bioreactor culture system has been demonstrated, confirming the ability of the scaffold to support cell colonisation and differentiation in a three-dimensional culture system.

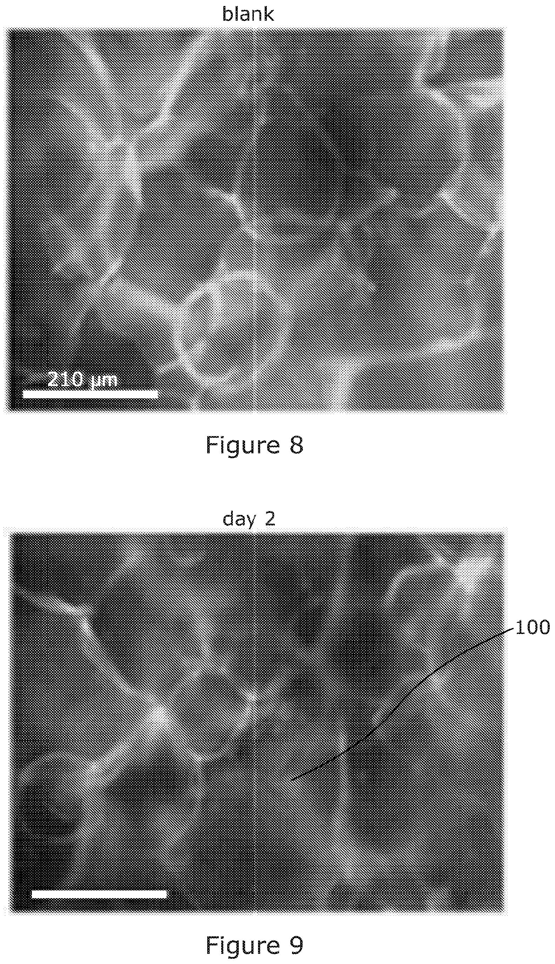

Figures 8 to 12 are fluorescent microscope images during the progress of a cytocompatibility test carried out on a chitosan-based scaffold according to an embodiment of the invention. The scale bar is 250 µm in each image. The cytocompatibility test was carried out *in vitro* using human mesenchymal stem cells.

Figure 8 shows the scaffold prior to cell seeding.

Figure 9 shows the scaffold two days after cell seeding. The stem cells show up as green in Figure 9. One of the areas where stem cells have grown within the scaffold is labelled 100.

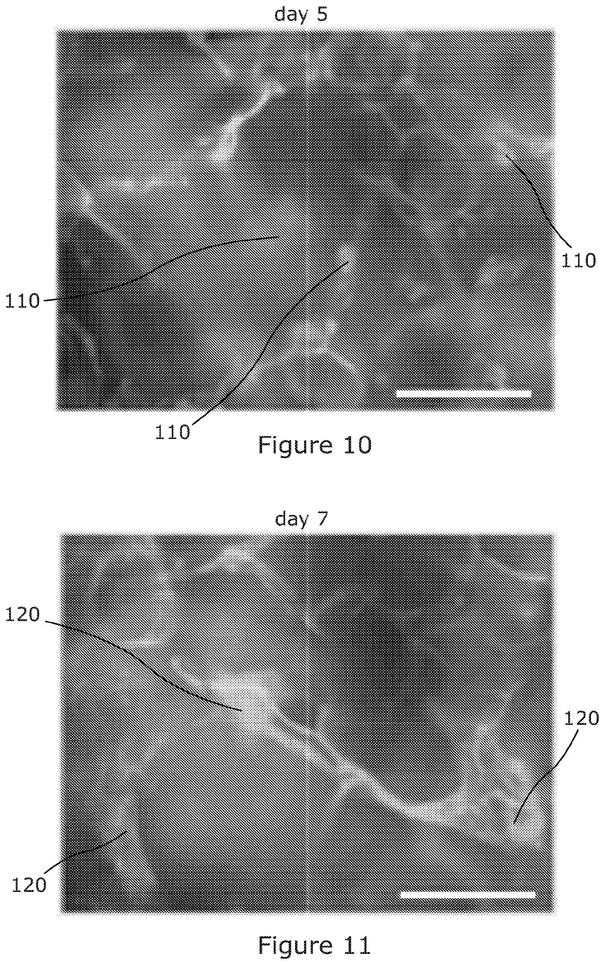

Figure 10 shows the scaffold five days after cell seeding. The stem cells show up as green in Figure 10. Some of the areas where stem cells have grown within the scaffold are labelled 110.

Figure 11 shows the scaffold seven days after cell seeding. The stem cells show up as green in Figure 11. Some of the areas where stem cells have grown within the scaffold are labelled 120.

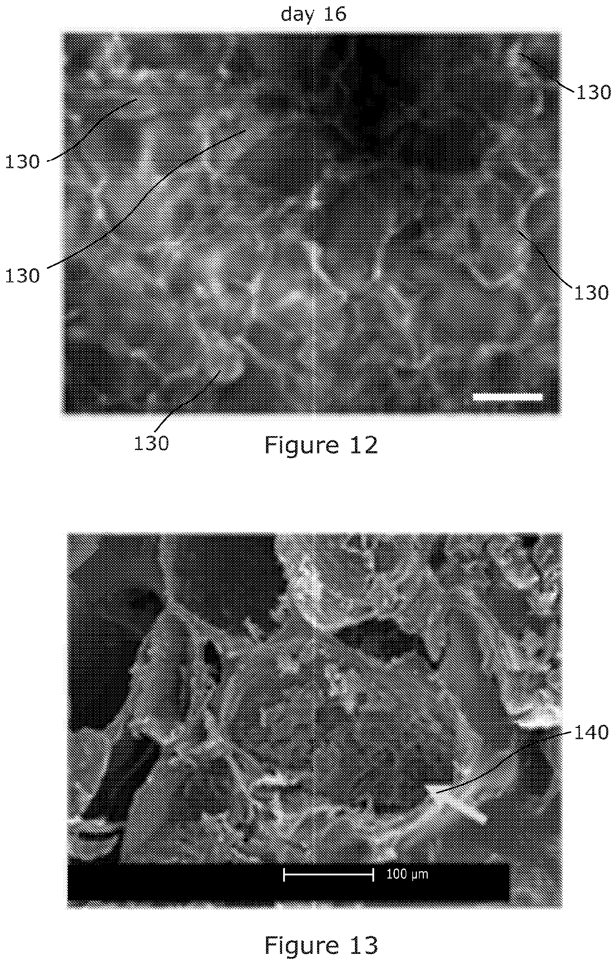

Figure 12 shows the scaffold 16 days after cell seeding. The stem cells show up as green in Figure 12. Some of the areas where stem cells have grown within the scaffold are labelled 130.

Thus, figures 8 to 12 illustrate the attachment, growth and colonisation of stem cells (human mesenchymal progenitors) within the scaffold over time.

Figure 13 is a scanning electron microscope (SEM) image of a cultured scaffold showing that human mesenchymal stem cells have adhered to and colonised the porous scaffold (as indicated, for instance, by label 140).

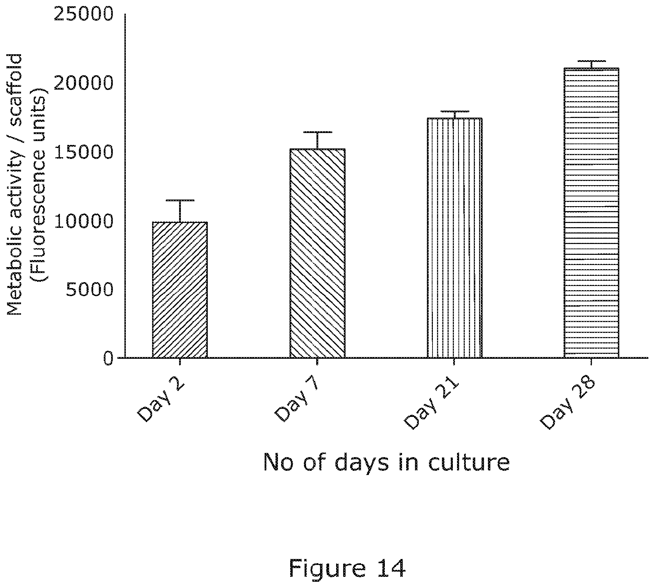

Figure 14 shows the results of cell metabolic activity analysis carried out on the scaffold during the cytocompatibility assessment. Fluorescence was used to detect metabolic activity. An increase in cellular metabolic activity over time in culture was observed.

The *in vitro* evaluations carried out indicate that the porous material and articles such as scaffolds comprising the porous material may offer superior structural and biomechanical qualities.

Conveniently, the invention may provide in some embodiments a non-delaminating porous construct for osteochondral repair. The porous construct may be a chitosan-based porous construct.

In some embodiments, the porous construct may have only one layer, i.e. there may be no predetermined spatial variation in porosity, average pore size or pore size distribution. The porous construct may comprise a plurality of layers, e.g. two, three, four, five, six, seven, eight, nine or 10 layers. The porous construct may comprise up to hundreds of layers, e.g. up to 100 layers, up to 200 layers or up to 500 layers.

The invention may provide porous materials according to the invention and/or articles comprising porous materials according to the invention having a controllable and/or graded pore size within at least one portion of the material or article, e.g. through a depth of the material or article.

The material according to the invention is manufactured using a combination of porogen leaching-out and freeze drying or solvent phase separation techniques.

Non-water-soluble polymer microspheres may serve as porogen to control the pore sizes. Typically, the pores may be generally spherical in form and may, in some embodiments, have a diameter of at least 100 µm and/or up to 150 µm.

The porogen microspheres may comprise polycaprolactone and/or polylactic acid. The porogen microspheres may comprise one or more polymers that can be removed in alkaline solutions. Polycaprolactone and polylactic acid are examples of polymers that can be removed in alkaline solutions.

The porogen microspheres may have diameters in the range of at least 50 µm and/or up to 500 µm. For example, the porogen microsphers may have diameters of up to or at least 180 µm and/or up to or at least 425 µm.

Typically, the porogen microspheres may be removed after the freeze drying or solvent phase separation step by treatment in alkaline alcohol solution at 50°C. The alkaline alcohol solution may comprise potassium hydroxide and ethanol or methanol.

The material may be exposed to, e.g. immersed in, the alkaline alcohol solution at an elevated temperature for a predetermined period of time. The predetermined period of time may be up to or at least 5 minutes, up to or at least 15 minutes, up to or at least 30 minutes, up to or least 1 hour, up to or at least 2 hours, up to or at least 3 hours, up to or at least 4 hours, up to or at least 5 hours, and/or up to or at least 6 hours. The process of leaching out the porogen may be performed at room temperature or at lower and higher temperatures, but it will require shorter or longer times dependent on the temperature calculable by an Arrhenius relationship.

Since embodiments of the invention may be intended for biomedical uses, the native tissue structure may be imitated in designing them. For instance, embodiments of the invention may be intended for use in osteochondral repair, and the tissue structure of the joint may be imitated in designing them.

Advantageously, the manufacturing process of the invention may be cost-effective and/or scalable. For instance, 1000s of the scaffolds could be readily manufactured using typical laboratory facilities. Furthermore, articles having complex geometries can be produced, since the material can be cast in a mould having any shape. In addition, the material can be shaped, e.g. cut, when dry or when wet. Hence, for instance, a surgeon could cut the material to shape before, during or after insertion of a biomedical implant comprising the material into a (human or animal) patient's body.

In some embodiments, a bone-mimicking layer may incorporate up to or at least 70wt% of hydroxyapatite nanoparticles (nHA) to enhance mechanical and osteogenesis properties.

No delamination was seen for this product under wet or dry conditions which made it unique in comparison with known commercial counterparts. A method was developed to eliminate the dark blue colour, not visually desirable for biomedical applications, obtained as a secondary product of the crosslinking of chitosan without causing adverse effects on the properties of the final products. It is envisaged that this product could be delivered using minimally invasive surgery (arthroscopy).

In embodiments, the invention may provide a novel non-delaminating multi-layered degradable scaffold with spatially resolved variable sized pores, which may be able to mimic the 3D architecture defining the articular cartilage/bone interface.

Advantageously, spatial resolution may be achieved within a single scaffold, rather than by joining two separate structures, in order to ensure the integrity of the resulting product.

In accordance with the present invention, by getting the appropriate conditions right the layers can be laid down and a non-delaminating structure achieved by crosslinking the whole structure in one single step, e.g. post layer construction.

Advantageously, embodiments of the invention may be useful in the treatment of orthopaedic pathologies such as osteoarthritis.

In the biomedical field, a graded scaffold according to any embodiment of the invention may offer a non-delaminating and cost-effective solution, optionally without relying on animal-derived collagen. Further, the graded scaffold may be capable of being arthroscopically delivered, reducing infection risk and improving recovery. By providing a product combining biomimetic, biocompatible and/or biomechanical benefits with ease of implantability and low cost of production, the present invention is expected to address existing limitations for the patient, the clinician and for the health services involved.

Conveniently, in a multi-layered article, the layers may have different colours from each other. This may be useful, for example, in ensuring that the article is used in the correct orientation.

The invention may have utility in many other biomedical applications, including as a scaffold (e.g. a monolayer scaffold) for cartilage surgery, a scaffold for bone grafting or in vascular applications. In vascular applications, the article may be provided in the form of a tube.

It will be appreciated that the material and articles, e.g. medical devices, comprising the material described herein may be applicable for use with animals as well as human patients and may be used in veterinary practice, e.g. to treat cats, dogs, horses, rabbits and the like.

The invention may also have utility in various non-biomedical applications. An example of a potential non-biomedical application for the invention is in the filtration and/or purification of fluids, e.g. water.

While the invention has been described with reference to certain specific embodiments, various modifications will be apparent to the person skilled in the art without departing from the scope of the application as filed.

## Claims

1. A porous material comprising a substantially continuous matrix comprising an at-least-partially-cross-linked water-soluble polymer with pores therein and a plurality of regions of differing pore sizes, average pore sizes, pore size distributions and/or porosities, wherein each region has a desired porosity, including a desired pore density and/or pore size distribution.

2. A porous material according to claim 1, wherein the cross-linking of the water-soluble polymer is provided by a cross-linking agent, optionally wherein the cross-linking agent comprises a chemical cross-linking agent or a physical cross-linking agent, optionally wherein the cross-linking agent comprises glutaraldehyde, formaldehyde, genipin, glyoxal, dextran sulfate, 1,1,3,3-tetramethoxypropane, oxidized cylclodextrins, ethylene glycoldiglyceryl ether, ethylene glycol diglycidyl ether (EGDE), diisocyanate, pentasodium tripolyphosphate (TPP), sodium polyphosphate or calcium chloride.

3. A porous material according to any one of the preceding claims, wherein at least one of the following applies:
(i) the material is coloured at least in part;
(ii) the porous material comprises spatial variations in porosity, optionally wherein the spatial variations in porosity are predetermined;
(iii) the porous material has graded porosity; and
(iv) the regions, e.g. layers, within the porous material, have a porosity of at least 10% and/or up to 90%.

4. A porous material according to any one of the preceding claims, wherein the material is capable of returning to its original shape after being subjected to up to 40%, up to 50%, up to 70% or up to 80% or more compressive strain.

5. A porous material according to any one of the preceding claims, which when dry, has a compressive strength of up to or at least 0.05 MPa, up to or at least 0.1 MPa or up to or at least 0.2 MPa and/or, when dry, has a compressive modulus of up to or at least 0.5 MPa, up to or at least 1 MPa, up to or at least 2 MPa or up to or at least 5 MPa and/or the porous material, when wet, has a compressive strength of up to or at least 2 kPa, up to or at least 5 kPa or up to or at least 10 kPa and/or, when wet, has a compressive modulus of up to or at least 12 kPa, up to or at least 25 kPa, up to or at least 50 kPa or up to or at least 500 kPa, optionally wherein the material is capable of returning to its original shape after being subjected to up to 40%, up to 50%, up to 70% or up to 80% or more compressive strain.

6. A porous material according to any one of claims 1 to 5, wherein the water-soluble polymer comprises chitosan, collagen, hyaluronic acid, gelatine, agarose, alginate, polyvinyl alcohol, dextran, polyacrylic acid, polyethylene glycol, polyvinyl pyrrolidone, polyacrylamides, cellulose ethers, starch or starch-based derivatives.

7. A porous material according to any one of claims 1 to 6, wherein the pores have a longest dimension, e.g. diameter, of at least 100 µm and/or up to 500 µm.

8. An article comprising the porous material of any one of claims 1 to 7.

9. The article according to claim 8, wherein the article comprises a medical device or a portion thereof such as a scaffold for osteochondral repair, a scaffold for bone grafting, an implant, e.g. for drug delivery, or a vascular stent or tube.

10. The article according to claim 8 or claim 9, wherein at least some of the pores are loaded with a drug or other therapeutic agent and/or at least some of the pores contain cells.

11. The article according to claim 8, claim 9 or claim 10, wherein at least a portion of the article is coated and/or impregnated with one or more antimicrobial agents and/or biological agents and/or metals.

## Patentansprüche

1. Poröses Material, umfassend eine im Wesentlichen kontinuierliche Matrix, die ein zumindest teilweise vernetztes, wasserlösliches Polymer mit Poren darin und einer Vielzahl von Bereichen mit unterschiedlichen Porengrößen, durchschnittlichen Porengrößen und Porengrößenverteilungen und/oder Porositäten umfasst, wobei jeder Bereich eine gewünschte Porosität, einschließlich einer gewünschten Porendichte und/oder Porengrößenverteilung, aufweist.

2. Poröses Material nach Anspruch 1, wobei die Vernetzung des wasserlöslichen Polymers durch ein Vernetzungsmittel gewährleistet wird, wobei das Vernetzungsmittel wahlweise ein chemisches Vernetzungsmittel oder ein physikalisches Vernetzungsmittel umfasst, wobei das Vernetzungsmittel wahlweise Glutaraldehyd, Formaldehyd, Genipin, Glyoxal, Dextransulfat, 1,1,3,3-Tetramethoxypropan, oxidierte Cyclodextrine, Ethylenglykoldiglycerylether, Ethylenglykoldiglycidylether (EGDE), Diisocyanat, Pentanatriumtripolyphosphat (TPP), Natriumpolyphosphat oder Calciumchlorid umfasst.

3. Poröses Material nach einem der vorstehenden Ansprüche, wobei mindestens eines von Folgendem gilt:
(i) das Material ist zumindest teilweise gefärbt;
(ii) das poröse Material umfasst räumliche Variationen bei der Porosität, wobei die räumlichen Variationen wahlweise bei der Porosität vorbestimmt sind;
(iii) das poröse Material weist eine abgestufte Porosität auf, und
(iv) die Bereiche, z. B. Schichten, innerhalb des porösen Materials weisen eine Porosität von mindestens 10 % und/oder bis zu 90 % auf.

4. Poröses Material nach einem der vorstehenden Ansprüche, wobei das Material in der Lage ist, zu seiner ursprünglichen Form zurückzukehren, nachdem es einer Druckverformung von bis zu 40 %, bis zu 50 %, bis zu 70 % oder bis zu 80 % oder mehr ausgesetzt war.

5. Poröses Material nach einem der vorstehenden Ansprüche, das, wenn es trocken ist, eine Druckfestigkeit von bis zu oder mindestens 0,05 MPa, bis zu oder mindestens 0,1 MPa oder bis zu oder mindestens 0,2 MPa aufweist und/oder, wenn es trocken ist, es einen Kompressionsmodul von bis zu oder mindestens 0,5 MPa, bis zu oder mindestens 1 MPa, bis zu oder mindestens 2 MPa oder bis zu oder mindestens 5 MPa aufweist und/oder das poröse Material weist, wenn es nass ist, eine Druckfestigkeit von bis zu oder mindestens 2 kPa, bis zu oder mindestens 5 kPa oder bis zu oder mindestens 10 kPa auf, und/oder das Material weist, wenn es nass ist, einen Kompressionsmodul von bis zu oder mindestens 12 kPa, bis zu oder mindestens 25 kPa, bis zu oder mindestens 50 kPa oder bis zu oder mindestens 500 kPa auf, wobei es wahlweise in der Lage ist, zu seiner ursprünglichen Form zurückzukehren, nachdem es einer Druckverformung von bis zu 40 %, bis zu 50 %, bis zu 70 % oder bis zu 80 % oder mehr ausgesetzt war.

6. Poröses Material nach einem der Ansprüche 1 bis 5, wobei das wasserlösliche Polymer Chitosan, Kollagen, Hyaluronsäure, Gelatine, Agarose, Alginat, Polyvinylalkohol, Dextran, Polyacrylsäure, Polyethylenglykol, Polyvinylpyrrolidon, Polyacrylamide, Celluloseether, Stärke oder Stärkederivate umfasst.

7. Poröses Material nach einem der Ansprüche 1 bis 6, wobei die Poren eine längste Abmessung, z. B. einen Durchmesser, von mindestens 100 µm und/oder bis zu 500 µm aufweisen.

8. Gegenstand, der das poröse Material nach einem der Ansprüche 1 bis 7 umfasst.

9. Gegenstand nach Anspruch 8, wobei der Gegenstand eine medizinische Vorrichtung oder einen Teil davon, wie beispielsweise ein Gerüst für die osteochondrale Reparatur, ein Gerüst für die Knochentransplantation, ein Implantat, z. B. zur Medikamentenverabreichung, oder einen Gefäßstent oder -schlauch, umfasst.

10. Gegenstand nach Anspruch 8 oder Anspruch 9, wobei mindestens einige von den Poren mit einem Arzneimittel oder einem anderen therapeutischen Wirkstoff beladen sind und/oder mindestens einige von den Poren Zellen enthalten.

11. Gegenstand nach Anspruch 8, Anspruch 9 oder Anspruch 10, wobei mindestens ein Teil des Gegenstands beschichtet und/oder mit einem oder mehreren antimikrobiellen Wirkstoffen und/oder biologischen Wirkstoffen und/oder Metallen imprägniert ist.

## Revendications

1. Matériau poreux comprenant une matrice sensiblement continue comprenant un polymère hydrosoluble au moins partiellement réticulé, ayant des pores et une pluralité de régions de tailles de pores différentes, de tailles de pores moyennes, de distributions de tailles de pores et/ou de porosités, dans lequel chaque région présente une porosité souhaitée, incluant une densité de pores souhaitée et/ou une distribution de la taille des pores.

2. Matériau poreux selon la revendication 1, dans lequel la réticulation du polymère hydrosoluble est assurée par un agent de réticulation, éventuellement dans lequel l'agent de réticulation comprend un agent de réticulation chimique ou un agent de réticulation physique, éventuellement dans lequel l'agent de réticulation comprend du glutaraldéhyde, du formaldéhyde, du génipine, du glyoxal, du sulfate de dextran, du 1,1,3,3-tétraméthoxypropane, des cyclodextrines oxydées, de l'éther diglycérylique d'éthylène glycol, de l'éther diglycidylique d'éthylène glycol (EGDE), du diisocyanate, du tripolyphosphate pentasodique (TPP), du polyphosphate de sodium ou du chlorure de calcium.

3. Matériau poreux selon l'une quelconque des revendications précédentes, dans lequel au moins une des conditions suivantes s'applique :
(i) le matériau est coloré au moins en partie ;
(ii) le matériau poreux comprend des variations spatiales de porosité, éventuellement dans lequel les variations spatiales de porosité sont prédéterminées ;
(iii) le matériau poreux présente une porosité graduelle ; et
(iv) les régions, par exemple les couches, au sein du matériau poreux, présentent une porosité d'au moins 10 % et/ou jusqu'à 90 %.

4. Matériau poreux selon l'une quelconque des revendications précédentes, dans lequel le matériau est capable de reprendre sa forme initiale après avoir été soumis à une contrainte allant jusqu'à 40 %, jusqu'à 50 %, jusqu'à 70 % ou jusqu'à 80 % ou plus de déformation par compression.

5. Matériau poreux selon l'une quelconque des revendications précédentes, qui, à l'état sec, présente une résistance à la compression allant jusqu'à ou au moins 0,05 MPa, allant jusqu'à ou au moins 0,1 MPa ou allant jusqu'à ou au moins 0,2 MPa et/ou, À l'état sec, son module de compression est égal ou supérieur à 0,5 MPa, égal ou supérieur à 1 MPa, égal ou supérieur à 2 MPa ou égal ou supérieur à 5 MPa, et/ou le matériau poreux, lorsqu'il est humide, présente une résistance à la compression allant jusqu'à ou au moins 2 kPa, allant jusqu'à ou au moins 5 kPa ou allant jusqu'à ou au moins 10 kPa et/ou, à l'état humide, présente un module de compression allant jusqu'à ou au moins 12 kPa, jusqu'à ou au moins 25 kPa, jusqu'à ou au moins 50 kPa ou jusqu'à ou au moins 500 kPa, dans lequel le matériau est éventuellement capable de reprendre sa forme initiale après avoir été soumis à une contrainte allant jusqu'à 40 %, jusqu'à 50 % jusqu'à 70 % ou jusqu'à 80 % ou plus de déformation par compression.

6. Matériau poreux selon l'une quelconque des revendications 1 à 5, dans lequel le polymère hydrosoluble comprend du chitosane, du collagène, de l'acide hyaluronique, de la gélatine, de l'agarose, de l'alginate, de l'alcool polyvinylique, du dextrane, de l'acide polyacrylique, du polyéthylène glycol, de la polyvinylpyrrolidone, des polyacrylamides, des éthers de cellulose, de l'amidon ou des dérivés à base d'amidon.

7. Matériau poreux selon l'une quelconque des revendications 1 à 6, dans lequel les pores présentent une dimension la plus longue, par exemple un diamètre, d'au moins 100 µm et/ou jusqu'à 500 µm.

8. Article comprenant le matériau poreux selon l'une quelconque des revendications 1 à 7.

9. Article selon la revendication 8, dans lequel l'article comprend un dispositif médical ou une partie de celui-ci tel qu'un échafaudage pour la réparation ostéochondrale, un échafaudage pour la greffe osseuse, un implant, par exemple pour l'administration de médicaments, ou un stent ou un tube vasculaire.

10. Article selon la revendication 8 ou 9, dans lequel au moins certains pores sont chargés d'un médicament ou d'un autre agent thérapeutique et/ou au moins certains des pores contiennent des cellules.

11. Article selon la revendication 8, la revendication 9 ou la revendication 10, dans lequel au moins une partie de l'article est revêtue et/ou imprégnée d'un ou plusieurs agents antimicrobiens et/ou d'agents biologiques et/ou de métaux.
